(19) Europäisches Patentamt
European Patent Office
Office européen des brevets

(11) Numéro de publication: **0 469 992 B1**

# (12) FASCICULE DE BREVET EUROPEEN

(45) Date de publication de fascicule du brevet: **21.09.94**

(21) Numéro de dépôt: **91402145.6**

(22) Date de dépôt: **30.07.91**

(51) Int. Cl.5: **C07D 237/20**, C07D 237/14, C07D 237/12, C07D 237/04, C07D 401/12, C07D 403/12, A61K 31/50, C07D 451/02, C07D 451/04, C07D 487/04

## (54) Dérivés d'alkyl-6 pyridazine, procédé de préparation et compositions pharmaceutiques en contenant.

(30) Priorité: **31.07.90 FR 9009777**

(43) Date de publication de la demande:
**05.02.92 Bulletin 92/06**

(45) Mention de la délivrance du brevet:
**21.09.94 Bulletin 94/38**

(84) Etats contractants désignés:
**AT BE CH DE DK ES FR GB GR IT LI LU NL SE**

(56) Documents cités:
**EP-A- 0 072 726**
**EP-A- 0 074 863**
**EP-A- 0 117 171**

**CHEMICAL ABSTRACTS, vol. 110, no. 1, 2 janvier 1989, pages 42-43, abrégé no. 504g, Columbus, Ohio, US; G.A. PINNA et al.: "Synthesis and pharmacological study of 5-aryl-6-methyl-4,5-dihydropyridazin-3(2H)-ones and related 5-aryl-6-methylpyridazin-3(2H)-ones"**

(73) Titulaire: **ELF SANOFI**
**32-34, rue Marbeuf**
**F-75008 Paris (FR)**

(72) Inventeur: **Boigegrain, Robert**
**Cemin de Peret**
**F-34820 Assas (FR)**
Inventeur: **Brodin, Roger**
**26 Mas des Bruyères,**
**Avenue de Monsieur Teste**
**F-34070 Montpellier (FR)**
Inventeur: **Kan, Jean-Paul**
**7, rue du Vallon**
**F-34830 Clapiers (FR)**
Inventeur: **Olliero, Dominique**
**12 Allée Jeanne Bourgeois,**
**Hameau de l'Aiguelongue**
**F-34100 Montpellier (FR)**
Inventeur: **Wermuth, Camille-Georges**
**3, rue de la Côte d'Azur**
**F-67100 Strasbourg (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**JOURNAL OF MEDICINAL CHEMISTRY, vol. 30, no. 2, février 1987, pages 239-249, Washington DC, US; C.G. WERMUTH et al.: "Synthesis and structure-activity relationships of a series of aminopyridazine derivatives of gamma-aminobutyric acid acting as selective GABA-A antagonists"**

(74) Mandataire: **Gillard, Marie-Louise et al**
**Cabinet Beau de Loménie**
**158, rue de l'Université**
**F-75340 Paris Cédex 07 (FR)**

## Description

Depuis de nombreuses années, des dérivés de pyridazine ont été proposés en tant que médicaments, notamment actifs sur le système cardiovasculaire ou sur le système nerveux central.

En particulier, le brevet français 2 510 998 et le brevet européen 72 726 divulguent des dérivés de pyridazine diversement substitués sur le cycle pyridazinique et portant tous en position 3 un substituant aminé du type

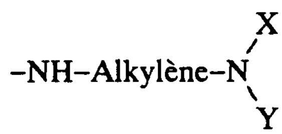

où X et Y représentent indépendamment l'hydrogène, un groupe alkyle ou forment avec l'atome d'azote auquel ils sont liés un hétérocycle tel que la morpholine.

Tous ces composés présentent une activité sur le système nerveux central en tant qu'antidépresseurs.

L'article J. Med. Chem., 1987, 30(239-249) décrit des antagonistes sélectifs des récepteurs du GABA, notamment la 6-méthyl-5-phényl-2H-pyridazin-3-one.

Selon la présente invention, on a maintenant trouvé qu'en modifiant les substituants du cycle pyridazinique, on obtient des composés ayant perdu leur activité antidépressive et ayant acquis une activité intéressante en tant que ligands des récepteurs cholinergiques de type $M_1$.

La présente invention a pour objet de nouveaux dérivés de pyridazine de formule :

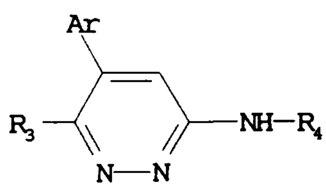

(I)

dans laquelle

- Ar représente un groupe phényle de formule

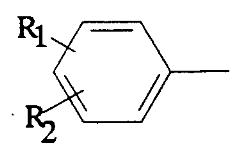

  un groupe pyridyle ou un groupe thiényle;
- $R_1$ et $R_2$ représentent chacun indépendamment l'hydrogène, un atome d'halogène, un groupe hydroxyle, un groupe alkyle en $C_1$-$C_4$, un alcoxy en $C_1$-$C_4$ ou le groupe trifluorométhyle;
- $R_3$ représente un alkyle droit ou ramifié en $C_1$-$C_5$, un groupe $-CH_2-C_6H_5$, un groupe $-CH_2-CH_2-C_6H_5$;
- $R_4$ représente le groupe

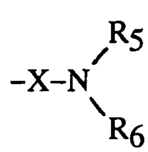

  dans lequel :
  - * X représente un alkylène droit ou ramifié en $C_1$-$C_6$ et notamment
    - . soit un groupe $-(CH_2)_n-$ dans lequel n est un nombre entier de 1 à 3;

. soit un groupe

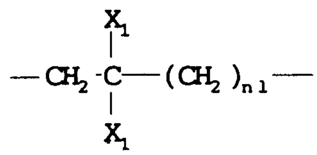

dans lequel $X_1$ est un groupe alkyle inférieur à $C_3$ lorsque $n_1 = 0$, ou un groupe méthyle lorsque $n_1 = 1$;

- $R_5$ et $R_6$ représentent chacun indépendamment l'hydrogène, un alkyle droit ou ramifié en $C_1$-$C_4$, ou constituent avec l'atome d'azote auquel ils sont liés, un hétérocycle choisi parmi la pyrrolidine, la morpholine ou la pipéridine, ou bien $R_4$ représente un groupe choisi parmi :

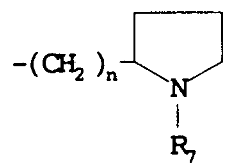

dans lequel n est tel que défini ci-dessus et $R_7$ représente l'hydrogène ou un groupe alkyle en $C_1$-$C_4$, de préférence en $C_1$-$C_2$;

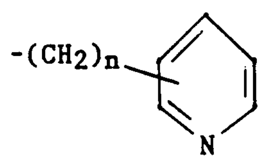

dans lequel n est tel que défini ci-dessus

ou

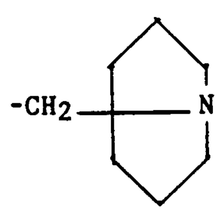

ou

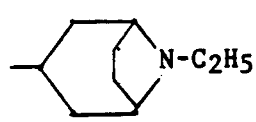

ou

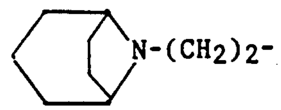

ainsi que leurs sels avec les acides minéraux ou organiques.

Les sels des composés de formule (I) selon la présente invention comprennent aussi bien ceux avec des acides minéraux ou organiques qui permettent une séparation ou une cristallisation convenable des composés de formule (I), tels que l'acide picrique ou l'acide oxalique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate.

Selon un autre de ses aspects, la présente invention concerne un procédé pour la préparation des composés de formule (I) caractérisé en ce que l'on fait réagir une chloro-3 pyridazine de formule

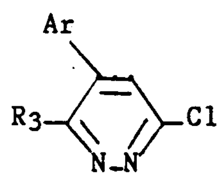

(II)

dans laquelle Ar et $R_3$ sont tels que définis précédemment, avec une amine de formule :

$R_4NH_2$

dans laquelle $R_4$ est tel que défini précédemment pour obtenir les composés (I) selon l'invention, et éventuellement on salifie le composé ainsi obtenu avec un acide minéral ou organique.

La réaction de substitution de la chloro-3 pyridazine (II) par l'amine $R_4NH_2$ est effectuée à une température comprise entre 100 et 150 °C, en l'absence de solvant ou en présence d'un solvant inerte tel que le n-butanol.

Le produit de formule (I) ainsi obtenu est isolé, sous forme de base libre ou de sel, selon les techniques conventionnelles.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2 sulfonate.

A la fin de la réaction, le composé de formule (I) peut être isolé sous forme d'un de ses sels, par exemple le chlorhydrate, l'oxalate ou le fumarate. Dans ce cas, s'il est nécessaire, la base libre peut être préparée par neutralisation du dit sel avec une base minérale ou organique telle que l'hydroxyde de sodium ou la triéthylamine ou avec un carbonate ou bicarbonate alcalin, tel que le carbonate ou bicarbonate de sodium ou de potassium.

Lorsque $R_1$ et/ou $R_2$ représentent un groupe hydroxyle, le composé selon l'invention est obtenu à partir du composé (I) dans lequel $R_1$ et/ou $R_2$ représentent un alcoxy, tous les autres substituants ayant les définitions ci-dessus, par déalkylation en utilisant des méthodes connues.

Les chloro-3 pyridazines (II), utilisées comme produits de départ, sont préparées à partir des 2H-pyridazinones-3 (III) de formule :

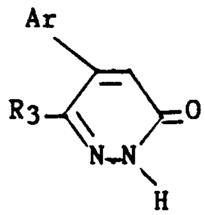

(III)

par réaction avec un excès d'oxychlorure de phosphore à chaud, sans solvant ou en présence d'un solvant inerte tel que l'acétonitrile.

Les 2H-pyridazinones-3 (III) ci-dessus sont obtenues par déshydrogénation des composés de formule (IV):

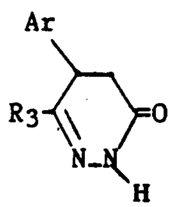

(IV)

par exemple, par action du brome dans de l'acide acétique.

Les tétrahydro-2,3,4,5 pyridazinones-3 de formule :

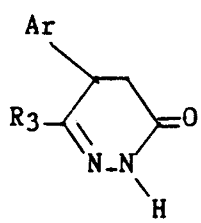

(IV)

et les dihydro-2,3 pyridazinones-3 de formule (III), font également partie de l'invention et sont préparées selon les méthodes décrites par SUTTER et al., J. Am. Chem. Soc., 1942, 64, 533-536, MUKHIRJI D., Science and Culture, 1948, 13, 426 et par PINNA G. et al., Il Farmaco, Ed. Sci., 1988, 43, 539-549.

Les composés intermédiaires utiles pour la préparation de (I), de formule :

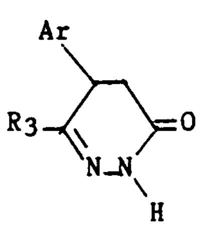

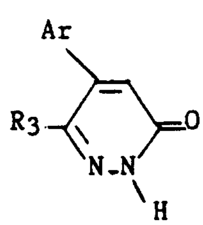

(IV) (III)

dans lesquels Ar et $R_3$ sont tels que définis précédemment, sont nouveaux et font partie de l'invention, à l'exception des composés pour lesquels Ar représente un phényle non substitué et $R_3$ un groupe méthyle. A titre d'exemple, des composés de formule (IV) et (III) sont décrits dans les tableaux 1 et 2 ci-après.

Les composés chlorés de formule (II)

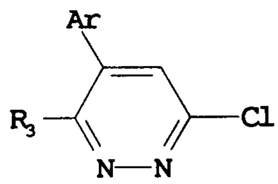

(II)

sont nouveaux et font partie de l'invention à l'exception du composé pour lequel Ar représente un groupe phényle et $R_3$ représente un groupe méthyle ; ils sont préparés selon les méthodes habituelles comme indiqué ci-dessus. A titre d'exemple, des composés de formule (II) sont décrits dans le tableau 3 ci-après.

La subtitution de ces composés chlorés (II) par une amine permet l'obtention des composés (I) dont le schéma réactionnel ci-dessous illustre la préparation.

SCHEMA 1

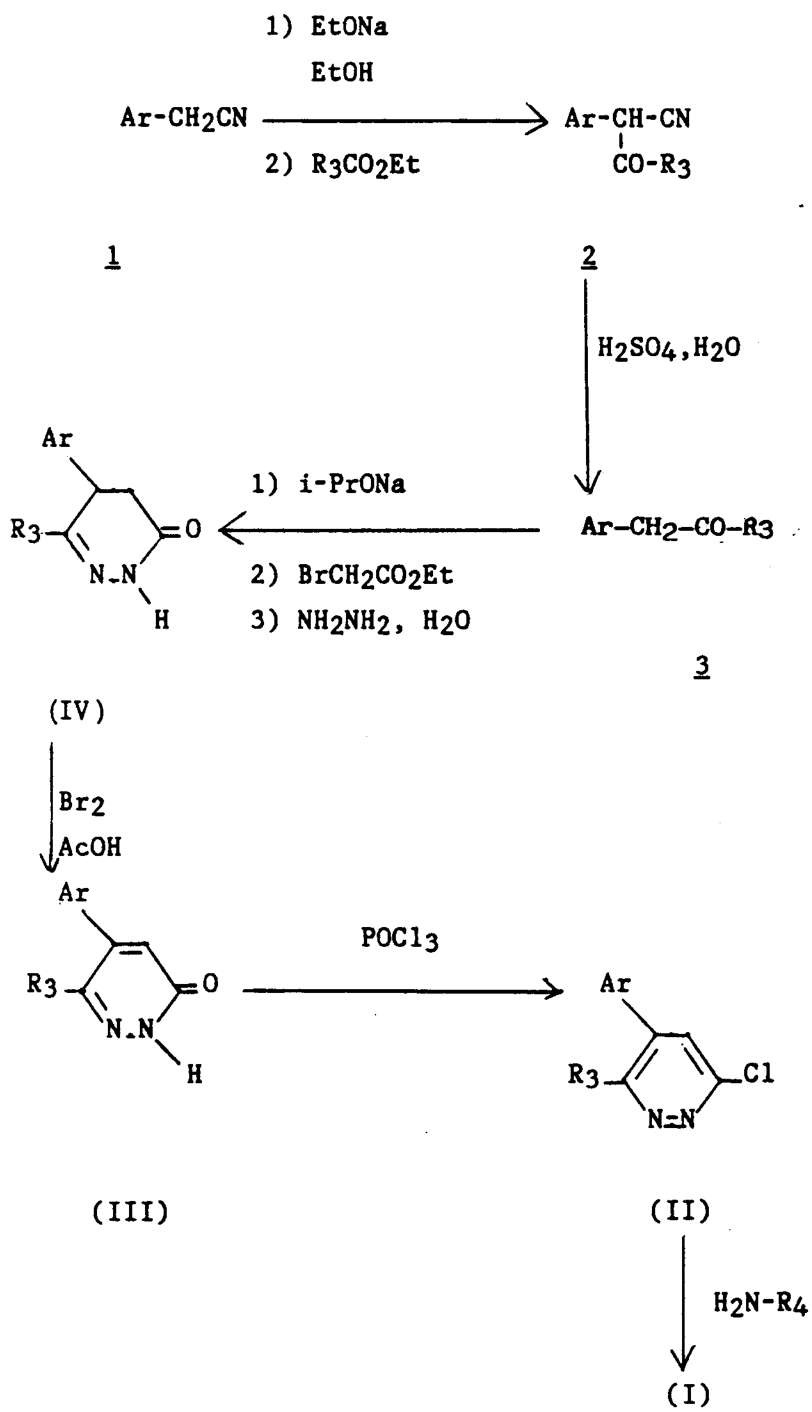


A partir d'un nitrile 1, on obtient par chauffage en présence d'une base et d'un dérivé ester carboxylique $R_3CO_2Et$, le $\beta$-cétonitrile 2, qui est transformé en milieu acide en dérivé cétonique 3 correspondant.

La cyclisation en tétrahydro-2,3,4,5 pyridazinone-3 est réalisée par réaction avec le bromoacétate d'éthyle dans un solvant alcoolique à température ambiante suivie de l'action de l'hydrate d'hydrazine à chaud.

La déshydrogénation du composé (IV) par action du brome dans l'acide acétique permet la préparation de la dihydro-2,3 pyridazinone-3 (III).

Dans le cas où $n_1$ = 0, les amines

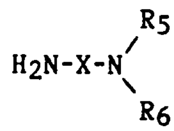

pour lesquelles X représente un groupe

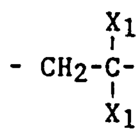

dans lequel $X_1$ est tel que défini précédemment sont préparées à partir d'un dérivé cyané de formule :

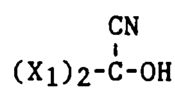

sur lequel on fait réagir une amine de formule $HNR_5R_6$ en présence éventuellement d'un sel d'acide fort tel que le sulfate de sodium ou de magnésium, à une température comprise entre 40 et 80°C pour obtenir l'aminonitrile de formule :

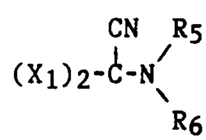

qui est hydraté selon les conditions habituelles en milieu acide pour fournir l'amide correspondant de formule :

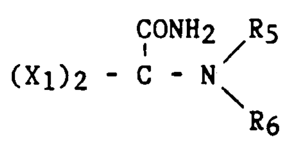

qui est alors réduit par action d'un hydrure métallique tel que l'hydrure de lithium aluminium ou l'hydrure de bore pour fournir l'amine attendue

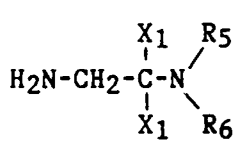

Les exemples suivants illustrent l'invention sans toutefois la limiter.

EXEMPLE 1

Dichlorhydrate de (diéthylamino-2 méthyl-2 propyl-1) amino-3 (fluoro-4 phényl)-5 propyl-6 pyridazine.

A) α butyryl fluoro-4 phénylacétonitrile.

10,9 g de sodium sont introduits dans 150 ml d'éthanol absolu. On ajoute, en maintenant le reflux, 49,5 g de fluoro-4 phénylacétonitrile en solution dans 65,2 g de butyrate d'éthyle. Le mélange réactionnel est chauffé à reflux pendant 4 heures puis laissé une nuit à température ambiante. Les solvants sont concentrés sous vide, le résidu est repris dans l'eau et lavé à l'éther. La phase aqueuse est refroidie dans un bain de glace et acidifiée à pH 5-6 par addition d'acide acétique concentré. On obtient alors des cristaux qui sont séparés par filtration, lavés à l'eau, dissous dans le dichlorométhane.

La phase organique est décantée, séchée sur $MgSO_4$, filtrée et concentrée sous vide.

m = 50 g

F = 53-54 °C.

B) (Fluoro-4 phényl)-1 pentanone-2.

50 g du produit obtenu précédemment sont traités par 36 ml d'eau et 148 ml d'acide sulfurique concentré qui est additionné goutte à goutte sous agitation à 4 °C. Le mélange est alors chauffé à 80 °C pendant 1/2 heure, refroidi, puis on additionne 534 ml d'eau et chauffe à reflux pendant une nuit.

Le mélange est extrait au dichlorométhane, la phase organique est décantée, séchée sur $Na_2SO_4$, filtrée et concentrée sous vide.

Le résidu est chromatographié sur gel de silice, éluant : cyclohexane/chlorure de méthylène : 50/50.

m = 39,2 g.

C) (Fluoro-4 phényl)-5 propyl-6 tétrahydro-2,3,4,5 pyridazinone-3.

2,76 g de sodium sont dissous dans 115 ml d'isopropanol. On additionne, en refroidissant dans un bain de glace, 21,56 g du produit obtenu précédemment en solution dans 30 ml d'isopropanol. Le mélange est agité pendant une heure à température ambiante puis on ajoute goutte à goutte 15 ml de bromoacétate d'éthyle en solution dans 20 ml d'isopropanol et on abandonne à température ambiante pendant 24 heures.

Le résidu est repris dans l'eau acide (HCl - pH = 2) et extrait au dichlorométhane. La phase organique est décantée et séchée sur $MgSO_4$.On filtre et concentre sous vide. Le résidu est repris dans 75 ml de butanol, on ajoute 11,25 ml d'hydrate d'hydrazine et chauffe le mélange réactionnel à reflux pendant 4 heures.

Le mélange est concentré sous vide, le résidu est repris dans l'eau et extrait au dichlorométhane. La phase organique est décantée, séchée sur $MgSO_4$, filtrée et concentrée sous vide.

m = 13,7 g.

F = 78-79 °C

D) Dihydro-2,3 (fluoro-4 phényl)-5 propyl-6 pyridazinone-3.

9,37 g du produit obtenu précédemment sont mis en solution dans 40 ml d'acide acétique et chauffés à 80 °C. On ajoute alors goutte à goutte 7,6 g de brome en solution dans 70 ml d'acide acétique et chauffe le mélange réactionnel à reflux pendant 4 heures. On refroidit à 15 °C et un précipité est séparé par filtration.

m = 6,6 g

F = 193-194 °C.

E) Chloro-3 (fluoro-4 phényl)-5 propyl-6 pyridazine.

4,64 g du produit obtenu précédemment sont dissous dans 28 ml d'acétonitrile et 8,6 ml d'oxychlorure de phosphore, le mélange réactionnel est chauffé à reflux pendant 4 heures puis concentré sous vide. Le résidu est repris dans l'eau glacée, additionnée d'ammoniaque puis on extrait au dichlorométhane. Les phases organiques sont décantées, séchées sur $MgSO_4$, filtrées et concentrées sous vide. Le résidu est chromatographié sur gel de silice, éluant : dichlorométhane/acétate d'éthyle 95/5.

m = 4,35 g
F = 64 °C.

F) Composé 1

1 g du produit obtenu précédemment et 3 g d'amino-1 diéthylamino-2 méthyl-2 propane sont chauffés à 120 °C, sous azote, pendant 48 heures.

Le mélange est concentré sous vide, le résidu est repris dans une solution de carbonate de sodium à 5 % et extrait au dichlorométhane. La phase organique est décantée, séchée sur $MgSO_4$, filtrée et concentrée sous vide. Le résidu est chromatographié sur gel de silice H Merck, éluant : dichlorométhane/méthanol 96/4.

Les fractions de produit pur sont concentrées sous vide, le résidu est repris dans l'éther et l'addition d'éther chlorhydrique permet la préparation du chlorhydrate qui est séparé par filtration.

m = 1,4 g
F = 100-115 °C.

EXEMPLES 2 A 19

A) En opérant comme il l'est décrit dans l'exemple 1, étape A, B et C, mais en remplaçant éventuellement le nitrile de départ par un autre dérivé nitrile et en faisant varier l'ester carboxylique, on obtient les tétrahydro-2,3,4,5 pyridazinones-3 répertoriées dans le tableau 1 ci-dessous.

TABLEAU 1

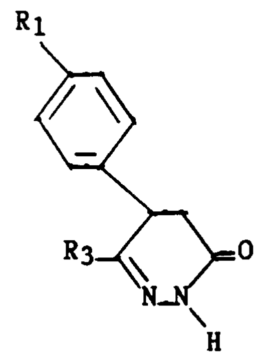

(IV)

| R1 | R3 | F ; °C | Solvant de recristallisation |
|---|---|---|---|
| H | $-CH_2CH_3$ | 131-132 | hexane |
| H | $n-C_3H_7$ | 104-105 | hexane |
| H | $i-C_3H_7$ | 137-138 | heptane |
| H | $i-C_4H_9$ | 92-93 | hexane |
| H | néopentyle | 155-156 | hexane |
| H | $-CH_2-C_6H_5$ | 114-115 | hexane |
| H | $-CH_2-CH_2-C_6H_5$ | 142-143 | $Et_2O$ |
| F | $n-C_3H_7$ | 78-79 | heptane |
| F | $i-C_4H_9$ | 106,5-107,5 | heptane |
| Cl | $-CH_3$ | 160-161 | $Et_2O$ |
| Cl | $-CH_2CH_3$ | 121-122 | hexane |
| Cl | $n-C_3H_7$ | 118-119 | heptane |

B) A partir des dérivés du tableau 1, on obtient, comme il l'est décrit dans l'exemple 1, étape D, par action du brome suivie d'une déshydrohalogénation, les dihydro-2,3 pyridazinones-3 répertoriées dans le tableau 2 ci-dessous. Ces composés ont tous été recristallisés d'un mélange dichlorométhane/hexane.

TABLEAU 2

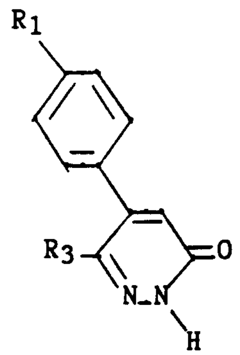

(III)

| $R_1$ | $R_3$ | F ; °C |
|---|---|---|
| H | $-CH_2CH_3$ | 123-124 |
| H | $n-C_3H_7$ | 162-163 |
| H | $i-C_3H_7$ | 178-179 |
| H | $i-C_4H_9$ | 154-155 |
| H | néopentyle | 181-182 |
| H | $-CH_2-C_6H_5$ | 140-141 |
| H | $-CH_2-CH_2-C_6H_5$ | 169-170 |
| F | $n-C_3H_7$ | 193-194 |
| F | $i-C_4H_9$ | 194-195 |
| Cl | $-CH_3$ | 212-214 |
| Cl | $-CH_2CH_3$ | 206-207 |
| Cl | $n-C_3H_7$ | 187-188 |

C) A partir des dérivés du tableau 2, on prépare les dérivés chlorés par action de l'oxychlorure de phosphore comme il l'est décrit dans l'exemple 1, étape E. Ces dérivés chlorés, répertoriés dans le tableau 3 ci-dessous ont tous été recristallisés du dichlorométhane.

TABLEAU 3

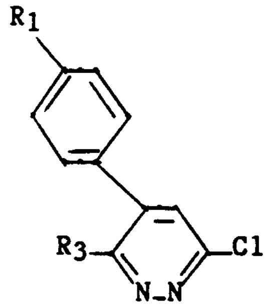

(II)

| R1 | R3 | F ; °C |
|---|---|---|
| H | $-CH_2CH_3$ | 49 |
| H | $n-C_3H_7$ | 58 |
| H | $i-C_3H_7$ | 86-87 |
| H | $i-C_4H_9$ | 70-71 |
| H | néopentyle | 99-100 |
| H | $-CH_2-C_6H_5$ | 99 |
| H | $-CH_2-CH_2-C_6H_5$ | 94-95 |
| F | $n-C_3H_7$ | 64 |
| F | $i-C_4H_9$ | huile |
| Cl | $-CH_3$ | 127-128 |
| Cl | $-CH_2CH_3$ | 84-85 |
| Cl | $n-C_3H_7$ | 63 |

D) La substitution des dérivés chlorés par une amine, selon l'exemple 1, étape F, permet la préparation des composés (I) selon l'invention, répertoriés dans les tableaux 4 , 5 et 6 ci-dessous.

TABLEAU 4

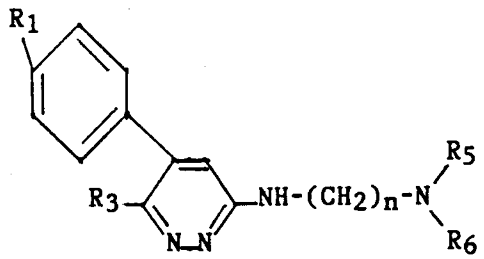

(I)

| exemple | $R_1$ | $R_3$ | n | 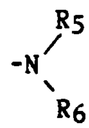 | F ; °C / Solvant recristallisation | Sel |
|---|---|---|---|---|---|---|
| 2 | H | $-CH_3$ | 2 | $-N-(C_2H_5)_2$ | 88 hexane | base |
| 3 | H | $n-C_3H_7$ | 2 | $-N-(C_2H_5)_2$ | 129 EtOH | fumarate |
| 4 | H | $-CH_3$ | 2 | 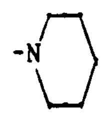 | 241 EtOH | 2HCl |
| 5 | H | $-CH_3$ | 2 | 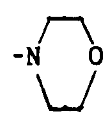 | 93 hexane | base |
| 6 | Cl | $n-C_3H_7$ | 3 | $-N-(C_2H_5)_2$ | 58 hexane | base |

TABLEAU 5

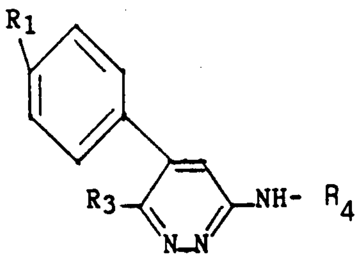

(I)

| exemple | $R_1$ | $R_3$ | $R_4$ | F ; °C<br>Solvant recristallisation<br>Sel |
|---|---|---|---|---|
| 7 | H | $-CH_3$ | 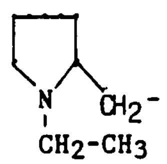 | 154 sesquifumarate<br>acétone |
| 8 | H | $n-C_3H_7$ | 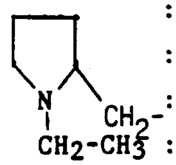 | 154 sesquifumarate<br>$Et_2O$ |
| 9 | H | $n-C_3H_7$ | 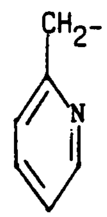 | 171 2HCl<br>$Et_2O$ |
| 10 | H | $-C_2H_5$ | 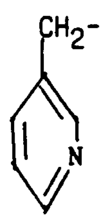 | 90 2HCl<br>$Et_2O$ |

TABLEAU 5 (Suite)

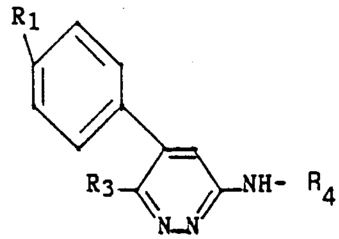

(I)

| exemple | $R_1$ | $R_3$ | $R_4$ | F ; °C<br>Solvant recristallisation<br>Sel | |
|---|---|---|---|---|---|
| 11 | H | $CH_3$ | 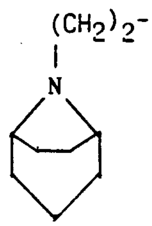 | 162,5<br>$Et_2O$ | difumarate |
| 12 | H | $CH_3$ | 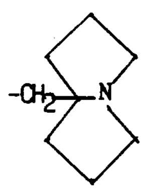 | 169,2<br>iPrOH | trifumarate |
| 13 | H | $i\text{-}C_4H_9$ | 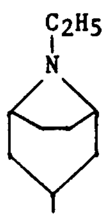<br>axiale | 99<br>$Et_2O$-iPrOH | difumarate<br>hémihydrate |
| 14 | H | $i\text{-}C_4H_9$ | 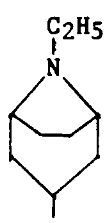<br>équatoriale | 151<br>$Et_2O$-iPrOH | difumarate |

TABLEAU 6

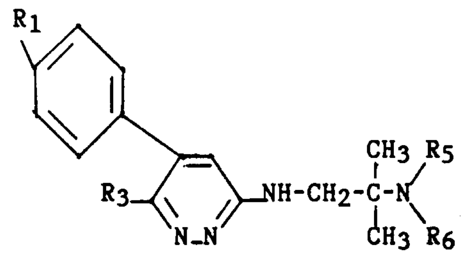

| exemple | $R_1$ | $R_3$ | 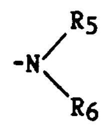 | F ; °C<br>Solvant recristallisation<br>Sel |
|---|---|---|---|---|
| 15 | H | $-CH_3$ | $-N-(C_2H_5)_2$ | 132-134 fumarate $1/2H_2O$<br>acétone |
| 16 | H | $-C_2H_5$ | $-N-(C_2H_5)_2$ | 95 difumarate<br>$Et_2O$ |
| 17 | H | $n-C_3H_7$ | $-N-(C_2H_5)_2$ | 121 difumarate<br>$Et_2O$ |
| 18 | H | $i-C_3H_7$ | $-N(C_2H_5)_2$ | 164-165 sesquifumarate<br>i-PrOH |
| 19 | H | $i-C_4H_9$ | $-N(C_2H_5)_2$ | 148-149 difumarate<br>$Et_2O$ |
| 20 | H | 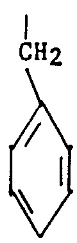 | $-N-(C_2H_5)_2$ | 115 2HCl<br>$Et_2O$ |

| | | | | | |
|---|---|---|---|---|---|
| 21 | H | $-CH_2$<br>\|<br>t-Bu | $-N(C_2H_5)_2$ | 114-115<br>$Et_2O$ | 2HCl |
| 22 | H | $-(CH_2)_2$<br>\|<br>$C_6H_5$ | $-N(C_2H_5)_2$ | 110<br>$Et_2O$ | 2HCl |
| 23 | F | $i-C_4H_9$ | $-N-(C_2H_5)_2$ | 161-162<br>$Et_2O$ | sesquifumarate |
| 24 | Cl | $-CH_3$ | $-N-(C_2H_5)_2$ | 100-105<br>$Et_2O$ | 2HCl |
| 25 | Cl | $-C_2H_5$ | $-N-(C_2H_5)_2$ | 95-100<br>$Et_2O$ | 2HCl |
| 26 | Cl | $n-C_3H_7$ | $-N-(C_2H_5)_2$ | 134-135<br>$Et_2O$ | difumarate |

Les propriétés pharmacologiques des composés selon l'invention ont été étudiées et en particulier leur affinité pour les récepteurs cholinergiques muscariniques de type $M_1$ et $M_2$.

In vitro, les composés (I) ont été essayés suivant la technique décrite par L. POTTER et al. J. Pharmacol. Exp. Ther., 1989, 284, 974-978 en ce qui concerne leur affinité pour les récepteurs de type $M_1$ et selon la technique décrite par HAMMER R. et al. Life Science, 1986, *38*, 1653-1662, en ce qui concerne leur affinité pour les récepteurs de type $M_2$.

Les composés selon l'invention présentent une bonne affinité pour les récepteurs de type $M_1$ et une spécificité marquée pour les récepteurs centraux de type $M_1$ vis-à-vis des récepteurs de type $M_2$.

A titre d'exemple, le composé 6 a montré une concentration inhibitrice 50 exprimée en micromole de 0,16 et 1,5 respectivement sur les récepteurs $M_1$ et $M_2$.

De même, le composé 25 a montré des concentrations inhibitrices 50 de 0,04 et 0,9 respectivement sur les récepteurs $M_1$ et $M_2$.

In vivo, les composés selon l'invention ont été essayés sur le test des rotations induites par la pirenzépine intrastriatale décrit par Worms P. et al. Psychopharmacology, 1987, *93*, 489-493.

A la dose de 3 mg par kg de poids corporel, per os, les produits selon l'invention inhibent fortement le nombre des rotations induites par la pirenzépine. Ainsi à titre d'exemple, le composé 6 inhibe de 71 % les rotations induites par la pirenzépine.

De plus les composés selon l'invention se sont montrés actifs sur le déficit mnésique induit par la scopolamine et le pentétrazole dans le test d'évitement passif chez le rat décrit par WORMS P. et al., Psychopharmacol., 1989, 98, 286-288.

Ainsi, d'après les résultats de ce test, le composé 6 selon l'invention s'oppose à l'amnésie induite par la pirenzépine administrée par voie intrapéritonéale à 75 mg/kg avec une $DE_{50}$ de 0,47 mg/kg po.

Enfin les composés selon l'invention n'ont donné aucun signe de toxicité aux doses où ils sont actifs.

Par suite les composés (I) peuvent être utilisés en tant que médicaments.

Les résultats indiqués montrent que les composés selon l'invention présentent une bonne affinité pour les récepteurs muscariniques et une bonne activité dans les tests d'amnésie induite par la scopolamine ou la pirenzépine. Ils permettent d'envisager l'utilisation des produits selon l'invention dans tous les cas où se manifeste un déficit cholinergique et notamment pour le traitement des troubles mnésiques, cognitifs, des syndromes dégénératifs liés à la sénescence et aux démences séniles.

Selon un autre de ses aspects la présente demande concerne donc les compositions pharmaceutiques contenant au moins un des composés de formule (I) ou un de leurs sels en tant que principe actif.

Dans les compositions pharmaceutiques de la présente invention pour l'administration orale, sublinguale, transdermique ou rectale, les principes actifs de formule (I) ci-dessus peuvent être administrés sous forme unitaire d'administration, en mélange avec les supports pharmaceutiques classiques, aux êtres humains notamment pour le traitement des troubles mnésiques cognitifs ou des syndromes dégénératifs. Les formes unitaires d'administration appropriées comprennent les formes par voie orale, telles que les comprimés, les gélules, les poudres, les granulés et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 5 et 1000 mg par jour.

Chaque dose unitaire peut contenir de 5 à 200 mg d'ingrédient actif en combinaison avec un support pharmaceutique. Cette dose unitaire peut être administrée 1 à 5 fois par jour.

Lorsqu'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans les gélules molles ou dures.

Les poudres ou les granulés dispersibles dans l'eau peuvent contenir le principe actif en mélange avec des agents de dispersion ou des agents mouillants, ou des agents de mise en suspension comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol et le butylèneglycol.

Le principe actif peut être formulé également sous forme de microcapsules, éventuellement avec un ou plusieurs supports ou additifs.

A titre de préparation galénique on peut préparer des gélules contenant ;

| | |
|---|---|
| Composé 6 | 0,010 g |
| Lactose | 0,050 g. |
| Stéarate de magnésium | 0,005 g. |

en mélangeant intimement les ingrédients ci-dessus et en versant le mélange dans des gélules de gélatine dure.

## Revendications

**Revendications pour les Etats contractants suivants : AT, BE, CH, DE, DK, FR, GB, IT, LI, LU, NL, SE**

**1.** Composé de formule

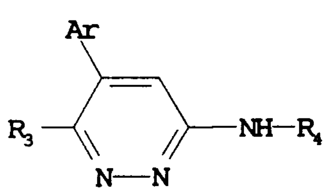

(I)

dans laquelle

- Ar représente un groupe phényle de formule

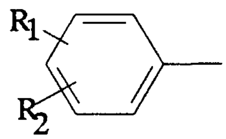

un groupe pyridyle ou un groupe thiényle;
- $R_1$ et $R_2$ représentent chacun indépendamment l'hydrogène, un atome d'halogène, un groupe hydroxyle, un groupe alkyle en $C_1$-$C_4$, un alcoxy en $C_1$-$C_4$ ou le groupe trifluorométhyle;
- $R_3$ représente un alkyle droit ou ramifié en $C_1$-$C_5$, un groupe $-CH_2-C_6H_5$, un groupe $-CH_2-CH_2-C_6H_5$;
- $R_4$ représente le groupe

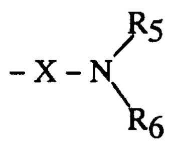

dans lequel :
. X représente un alkylène droit ou ramifié en $C_1$-$C_6$;
. $R_5$ et $R_6$ représentent chacun indépendamment l'hydrogène, un alkyle droit ou ramifié en $C_1$-$C_4$, ou constituent avec l'atome d'azote auquel ils ont liés, un hétérocycle choisi parmi la pyrrolidine, la morpholine ou la pipéridine,

ou bien $R_4$ représente un groupe chois parmi :

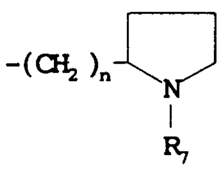

dans lequel n est un nombre entier de 1 à 3 et $R_7$ représente l'hydrogène ou un groupe alkyle en $C_1$-$C_4$;

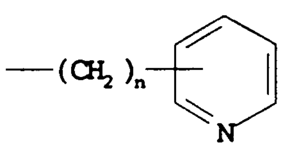

dans lequel n est tel que défini ci-dessus

ou

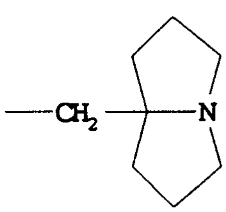

ou

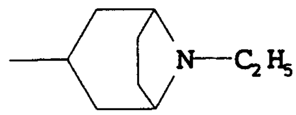

ou

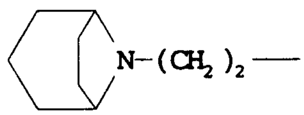

ainsi que ses sels avec les acides minéraux ou organiques.

**2.** Composé selon la revendication 1, caractérisé en ce que $R_4$ représente le groupe

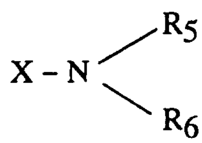

dans lequel
- X représente
  . soit un groupe $-(CH_2)_n-$ dans lequel n est un nombre entier de 1 à 3;
  . soit un groupe

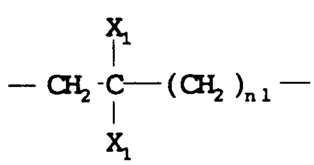

dans lequel $X_1$ est un groupe alkyle inférieur à $C_3$ lorsque $n_1 = 0$, ou un groupe méthyle lorsque $n_1 = 1$:
- $R_5$ et $R_6$ sont tels que définis dans la revendication 1.

**3.** Composé selon la revendication 1, caractérisé en ce que $R_7$ représente un groupe méthyle ou éthyle.

**4.** Composé intermédiaire pour la préparation du composé (I) selon la revendication 1, de formule :

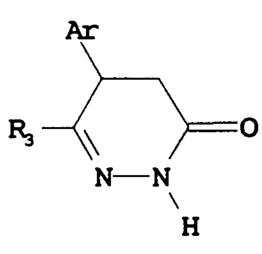

(IV)

dans laquelle Ar et $R_3$ sont tels que définis dans la revendication 1 à l'exception du composé (IV) pour lequel $R_1 = R_2 = H$ et $R_3 = CH_3$.

**5.** Composé intermédiaire pour la préparation du composé (I) selon la revendication 1, de formule :

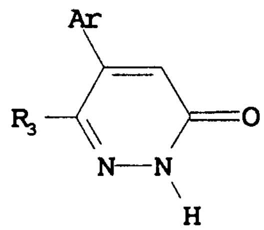 (III)

dans laquelle Ar et $R_3$ sont tels que définis dans la revendication 1 à l'exception du composé (III) pour lequel $R_1 = R_2 = H$ et $R_3 = CH_3$.

**6.** Composé intermédiaire pour la préparation du composé (I) selon la revendication 1, de formule :

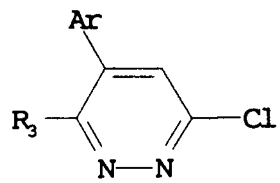 (II)

dans laquelle Ar et $R_3$ sont tels que définis dans la revendication 1 à l'exception du composé (II) pour lequel $R_1 = R_2 = H$ et $R_3 = CH_3$.

**7.** Procédé de préparation d'un composé selon la revendication 1 caractérisé en ce que l'on fait réagir une amine de formule :

$H_2N-R_4$

dans laquelle $R_4$ est tel que défini dans la revendication 1 avec un dérivé chloro-3 pyridazine de formule :

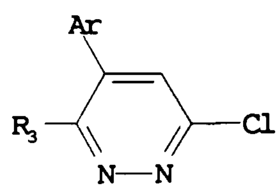 (II)

dans laquelle Ar et $R_3$ sont tels que définis dans la revendication 1 et éventuellement, on salifie le composé ainsi obtenu avec un acide minéral ou organique.

**8.** Composition pharmaceutique contenant, en tant que principe actif, un composé de formule (I) selon l'une quelconque des revendications 1 à 3.

**9.** Composition pharmaceutique selon la revendication 8 sous forme d'unité de dosage, dans laquelle le principe actif est mélangé à au moins un excipient pharmaceutique.

**Revendications pour l'Etats contractant suivant : ES**

**1.** Procédé pour l'obtention de dérivés d'alkyl-6-pyridazine de formule :

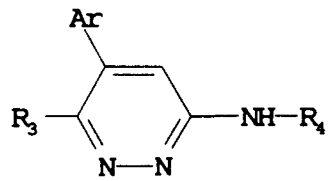

(I)

dans laquelle

- Ar représente un groupe phényle de formule

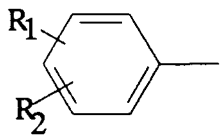

un groupe pyridyle ou un groupe thiényle;
- $R_1$ et $R_2$ représentent chacun indépendamment l'hydrogène, un atome d'halogène, un groupe hydroxyle, un groupe alkyle en $C_1$-$C_4$, un alcoxy en $C_1$-$C_4$ ou le groupe trifluorométhyle;
- $R_3$ représente un alkyle droit ou ramifié en $C_1$-$C_5$, un groupe $-CH_2-C_6H_5$, un groupe $-CH_2-CH_2-C_6H_5$;
- $R_4$ représente le groupe

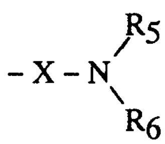

dans lequel :
. X représente un alkylène droit ou ramifié en $C_1$-$C_6$;
. $R_5$ et $R_6$ représentent chacun indépendamment l'hydrogène, un alkyle droit ou ramifié en $C_1$-$C_4$, ou constituent avec l'atome d'azote auquel ils ont liés, un hétérocycle choisi parmi la pyrrolidine, la morpholine ou la pipéridine,

ou bien $R_4$ représente un groupe chois parmi :

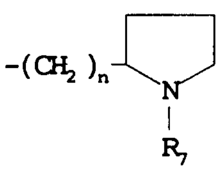

dans lequel n est un nombre entier de 1 à 3 et $R_7$ représente l'hydrogène ou un groupe alkyle en $C_1$-$C_4$;

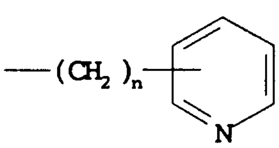

dans lequel n est tel que défini ci-dessus

ou

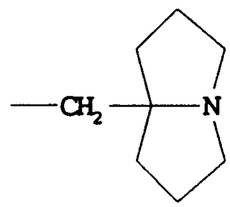

ou

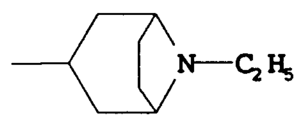

ou

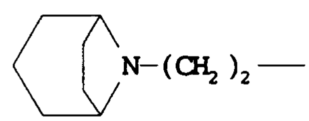

ainsi que ses sels avec les acides minéraux ou organiques, caractérisé en ce qu'il consiste à faire réagir une amine de formule :

$H_2N-R_4$

dans laquelle $R_4$ est tel que défini ci-dessus avec une chloro-3 pyridazine de formule :

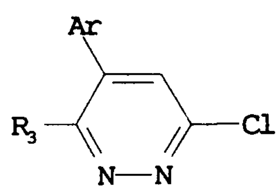

(II)

dans laquelle Ar et $R_3$ sont tels que définis ci-dessus, et éventuellement à salifier le composé ainsi obtenu avec un acide minéral ou organique.

**2.** Procédé selon la revendication 1, caractérisé en ce que l'on utilise une amine de formule $H_2N-R_4$ dans laquelle $R_4$ représente un groupe

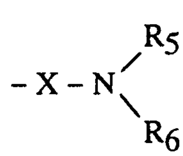

dans lequel

- X représente
  . soit un groupe $-(CH_2)_n-$ dans lequel n est un nombre entier de 1 à 3;

. soit un groupe

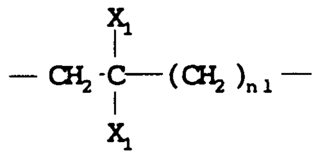

dans lequel $X_1$ est un groupe alkyle inférieur à $C_3$ lorsque $n_1 = 0$, ou un groupe méthyle lorsque $n_1 = 1$;

- $R_5$ et $R_6$ sont tels que définis dans la revendication 1.

**3.** Procédé selon la revendication 1, caractérisé en ce que l'on utilise une amine de formule $H_2N\text{-}R_4$ dans laquelle $R_4$ représente un groupe

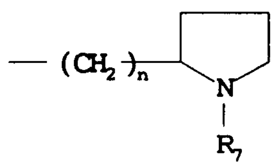

dans lequel n est un nombre entier de 1 à 3 et $R_7$ est un groupe méthyle ou éthyle.

**4.** Procédé pour l'obtention de composés de formule :

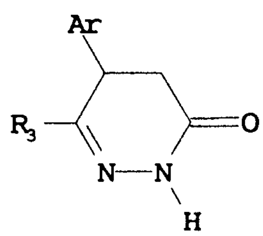

(IV)

dans laquelle :

- Ar représente un groupe phényle de formule

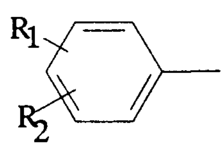

un groupe pyridyle ou un groupe thiényle;

- $R_1$ et $R_2$ représentent chacun indépendamment l'hydrogène, un atome d'halogène, un groupe hydroxyle, un groupe alkyle en $C_1$-$C_4$, un alcoxy en $C_1$-$C_4$ ou le groupe trifluorométhyle;
- $R_3$ représente un alkyle droit ou ramifié en $C_1$-$C_5$, un groupe $-CH_2\text{-}C_6H_5$, un groupe $-CH_2\text{-}CH_2\text{-}C_6H_5$;

à l'exception du compose (IV) pour lequel $R_1 = R_2 = H$ et $R_3 = CH_3$ ;

caractérisé en ce qu'il consiste à cycliser le dérivé cétonique de formule :

$Ar\text{-}CH_2\text{-}CO\text{-}R_3$

dans laquelle Ar et $R_3$ sont tels que définis ci-dessus par réaction avec le bromoacétate d'éthyle dans un solvant alcoolique à température ambiante, puis par réaction avec l'hydrate d'hydrazine à chaud.

5. Procédé pour l'obtention de composés de formule :

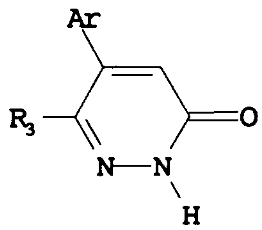 (III)

dans laquelle :

- Ar représente un groupe phényle de formule

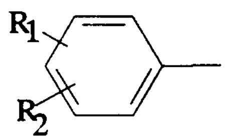

un groupe pyridyle ou un groupe thiényle;
- $R_1$ et $R_2$ représentent chacun indépendamment l'hydrogène, un atome d'halogène, un groupe hydroxyle, un groupe alkyle en $C_1$-$C_4$, un alcoxy en $C_1$-$C_4$ ou le groupe trifluorométhyle;
- $R_3$ représente un alkyle droit ou ramifié en $C_1$-$C_5$, un groupe $-CH_2-C_6H_5$, un groupe $-CH_2-CH_2-C_6H_5$;

à l'exception du composé (III) pour lequel $R_1 = R_2 = H$ et $R_3 = CH_3$ ;

caractérisé en ce qu'il consiste à déshydrogéner le composé de formule IV:

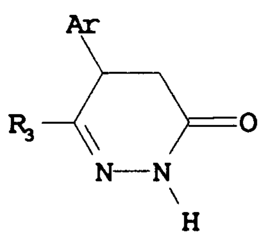 (IV)

dans laquelle Ar et $R_3$ sont tels que définis ci-dessus par action du brome dans de l'acide acétique.

6. Procédé pour l'obtention de composés de formule :

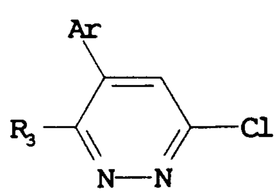 (II)

dans laquelle :

- Ar représente un groupe phényle de formule

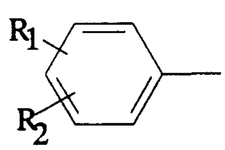

un groupe pyridyle ou un groupe thiényle;

- $R_1$ et $R_2$ représentent chacun indépendamment l'hydrogène, un atome d'halogène, un groupe hydroxyle, un groupe alkyle en $C_1$-$C_4$, un alcoxy en $C_1$-$C_4$ ou le groupe trifluorométhyle;
- $R_3$ représente un alkyle droit ou ramifié en $C_1$-$C_5$, un groupe $-CH_2-C_6H_5$, un groupe $-CH_2-CH_2-C_6H_5$;

à l'exception du composé (II) pour lequel $R_1 = R_2 = H$ et $R_3 = CH_3$ ;

caractérisé en ce qu'il consiste à faire réagir un composé de formule :

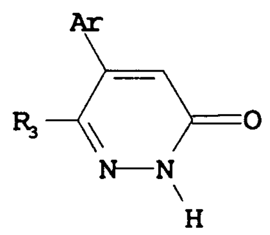

(III)

dans laquelle Ar et $R_3$ sont tels que définis ci-dessus avec un excès d'oxychlorure de phosphore à chaud, sans solvant ou en présence d'un solvant inerte tel que l'acétonitrile.

7. Procédé pour la préparation d'une composition pharmaceutique caractérisé en ce que l'on mélange, en tant que principe actif, un composé préparé selon le procédé de l'une quelconque des revendications 1 à 3 avec un véhicule pharmaceutiquement acceptable.

**Revendications pour l'Etat contractant suivant : GR**

1. Composé de formule

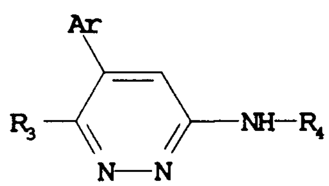

(I)

dans laquelle

- Ar représente un groupe phényle de formule

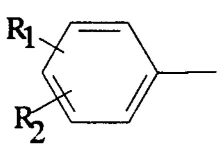

un groupe pyridyle ou un groupe thiényle;

- $R_1$ et $R_2$ représentent chacun indépendamment l'hydrogène, un atome d'halogène, un groupe hydroxyle, un groupe alkyle en $C_1$-$C_4$, un alcoxy en $C_1$-$C_4$ ou le groupe trifluorométhyle;
- $R_3$ représente un alkyle droit ou ramifié en $C_1$-$C_5$, un groupe $-CH_2-C_6H_5$, un groupe $-CH_2-CH_2-C_6H_5$;
- $R_4$ représente le groupe

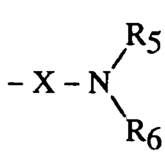

dans lequel :

. X représente un alkylène droit ou ramifié en $C_1$-$C_6$;

. $R_5$ et $R_6$ représentent chacun indépendamment l'hydrogène, un alkyle droit ou ramifié en $C_1$-$C_4$, ou constituent avec l'atome d'azote auquel ils ont liés, un hétérocycle choisi parmi la pyrrolidine, la morpholine ou la pipéridine,

ou bien $R_4$ représente un groupe choisi parmi :

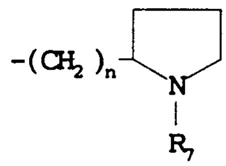

dans lequel n est un nombre entier de 1 à 3 et $R_7$ représente l'hydrogène ou un groupe alkyle en $C_1$-$C_4$;

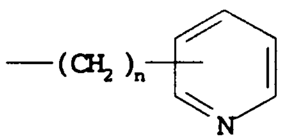

dans lequel n est tel que défini ci-dessus

ou

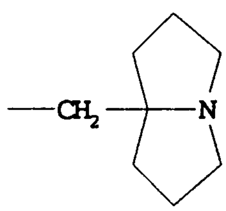

ou

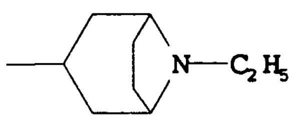

ou

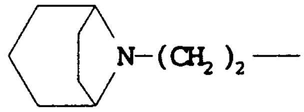

ainsi que ses sels avec les acides minéraux ou organiques.

**2.** Composé selon la revendication 1, caractérisé en ce que $R_4$ représente le groupe

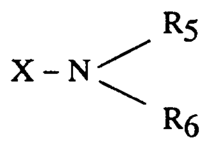

dans lequel

- X représente
  . soit un groupe $-(CH_2)_n-$ dans lequel n est un nombre entier de 1 à 3;
  . soit un groupe

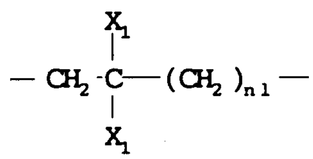

dans lequel $X_1$ est un groupe alkyle inférieur à $C_3$ lorsque $n_1 = 0$, ou un groupe méthyle lorsque $n_1 = 1$;

- $R_5$ et $R_6$ sont tels que définis dans la revendication 1.

**3.** Composé selon la revendication 1, caractérisé en ce que $R_7$ représente un groupe méthyle ou éthyle.

**4.** Composé intermédiaire pour la préparation du composé (I) selon la revendication 1, de formule :

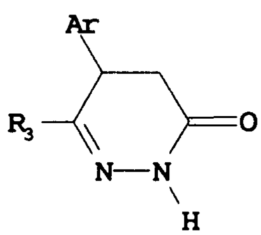

(IV)

dans laquelle Ar et $R_3$ sont tels que définis dans la revendication 1 à l'exception du composé (IV) pour lequel $R_1 = R_2 = H$ et $R_3 = CH_3$.

**5.** Composé intermédiaire pour la préparation du composé (I) selon la revendication 1, de formule :

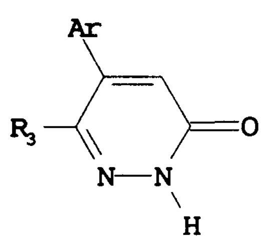

(III)

dans laquelle Ar et $R_3$ sont tels que définis dans la revendication 1 à l'exception du composé (III) pour lequel $R_1 = R_2 = H$ et $R_3 = -CH_3$.

**6.** Composé intermédiaire pour la préparation du composé (I) selon la revendication 1, de formule :

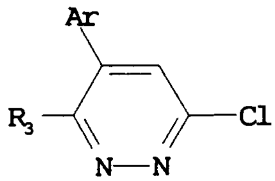

(II)

dans laquelle Ar et $R_3$ sont tels que définis dans la revendication 1 à l'exception du composé (II) pour lequel $R_1 = R_2 = H$ et $R_3 = CH_3$.

**7.** Procédé de préparation d'un composé selon la revendication 1 caractérisé en ce que l'on fait réagir une amine de formule :

$H_2N\text{-}R_4$

dans laquelle $R_4$ est tel que défini dans la revendication 1 avec un dérivé chloro-3 pyridazine de formule :

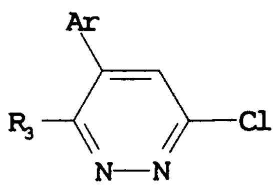

(II)

dans laquelle Ar et $R_3$ sont tels que définis dans la revendication 1 et éventuellement, on salifie le composé ainsi obtenu avec un acide minéral ou organique.

**8.** Procédé pour la préparation d'une composition pharmaceutique, caractérisé en ce que l'on mélange, en tant que principe actif, un composé selon l'une quelconque des revendications 1 à 3 avec un véhicule pharmaceutiquement acceptable.

**Claims**
**Claims for the following Contracting States : AT, BE, CH, DE, DK, FR, GB, IT, LI, LU, NL, SE**

**1.** Compound of the formula

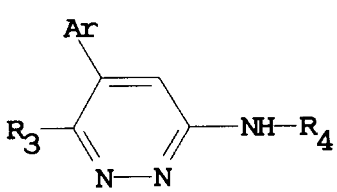

(I)

in which
- Ar is a phenyl group of the formula

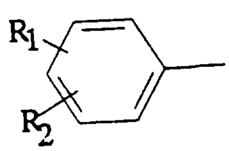

a pyridyl group or a thienyl group;

- $R_1$ and $R_2$ are each independently hydrogen, a halogen atom, a hydroxyl group, a $C_1$-$C_4$ alkyl group, a $C_1$-$C_4$ alkoxy or the trifluoromethyl group;
- $R_3$ is a linear or branched $C_1$-$C_5$ alkyl, a group $-CH_2-C_6H_5$ or a group $-CH_2-CH_2-C_6H_5$;
- $R_4$ is the group

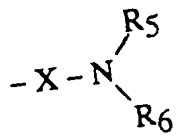

in which:

. X is a linear or branched $C_1$-$C_6$ alkylene;

. $R_5$ and $R_6$ are each independently hydrogen or a linear or branched $C_1$-$C_4$ alkyl or form, with the nitrogen atom to which they are bonded, a heterocycle selected from pyrrolidine, morpholine or piperidine

or $R_4$ is a group selected from:

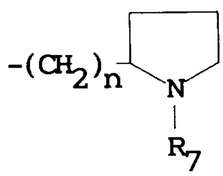

in which n is an integer from 1 to 3 and $R_7$ is hydrogen or a $C_1$-$C_4$ alkyl group;

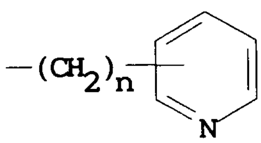

in which n is as defined above

or

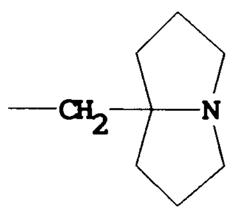

or

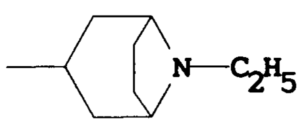

or

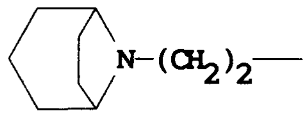

as well as its salts with mineral or organic acids.

**2.** Compound according to claim 1, characterized in that $R_4$ represents the group

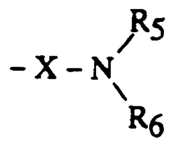

in which
- X represents
  - . either a group $-(CH_2)_n-$, in which n is an integer from 1 to 3;
  - . or a group

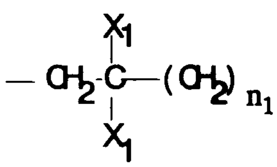

in which $X_1$ is an alkyl group which is lower than $C_3$ when $n_1 = 0$, or a methyl group when $n_1 = 1$;
- $R_5$ and $R_6$ are as defined in claim 1.

**3.** Compound according to claim 1, characterized in that $R_7$ is a methyl or ethyl group.

**4.** Intermediate compound for the preparation of compound (I) according to claim 1, of the formula:

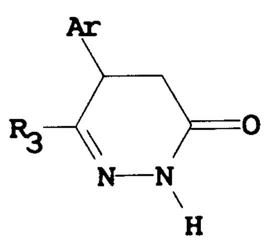

(IV)

in which Ar and $R_3$ are as defined in claim 1, with the exception of the compound (IV) in which $R_1 = R_2 = H$ and $R_3 = CH_3$.

**5.** Intermediate compound for the preparation of compound (I) according to claim 1, of the formula:

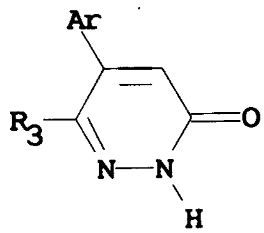

(III)

in which Ar and $R_3$ are as defined in claim 1, with the exception of the compound (III) in which $R_1 = R_2 = H$ and $R_3 = CH_3$.

**6.** Intermediate compound for the preparation of compound (I) according to claim 1, of the formula:

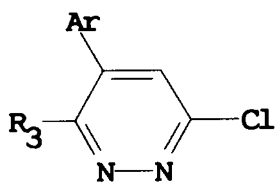

(II)

in which Ar and $R_3$ are as defined in claim 1, with the exception of the compound (II) in which $R_1 = R_2 = H$ and $R_3 = CH_3$.

**7.** Method of preparing a compound according to claim 1, characterized in that an amine of the formula

$H_2N\text{-}R_4$

in which $R_4$ is as defined in claim 1, is reacted with a 3-chloropyridazine derivative of the formula

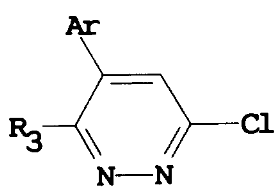

(II)

in which Ar and $R_3$ are as defined in claim 1, and, if desired, the resulting compound is converted to a salt with a mineral or organic acid.

**8.** Pharmaceutical composition in which a compound of formula (I) according to any one of claims 1 to 3 is present as the active principle.

**9.** Pharmaceutical composition according to claim 8 in the form of a dosage unit in which the active principle is mixed with at least one pharmaceutical excipient.

**Claims for the following Contracting State : ES**

**1.** Method for obtaining 6-alkylpyridazine derivatives of formula:

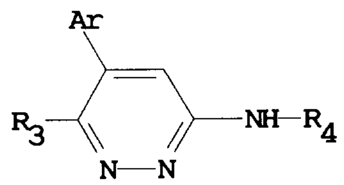

(I)

in which

- Ar is a phenyl group of the formula

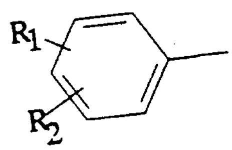

  a pyridyl group or a thienyl group;
- $R_1$ and $R_2$ are each independently hydrogen, a halogen atom, a hydroxyl group, a $C_1$-$C_4$ alkyl group, a $C_1$-$C_4$ alkoxy or the trifluoromethyl group;
- $R_3$ is a linear or branched $C_1$-$C_5$ alkyl, a group $-CH_2-C_6H_5$ or a group $-CH_2-CH_2-C_6H_5$;
- $R_4$ is the group

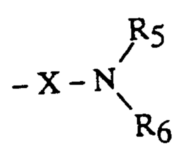

in which:

. X is a linear or branched $C_1$-$C_6$ alkylene;

. $R_5$ and $R_6$ are each independently hydrogen or a linear or branched $C_1$-$C_4$ alkyl or form, with the nitrogen atom to which they are bonded, a heterocycle selected from pyrrolidine, morpholine or piperidine

or $R_4$ is a group selected from:

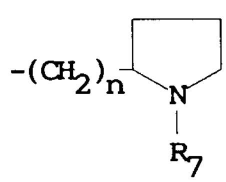

in which n is an integer from 1 to 3 and $R_7$ is hydrogen or a $C_1$-$C_4$ alkyl group;

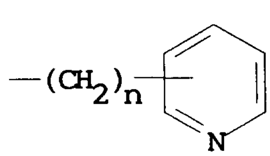

in which n is as defined above

or

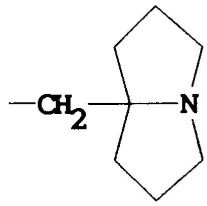

or

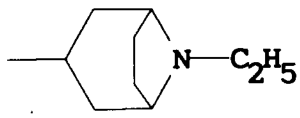

or

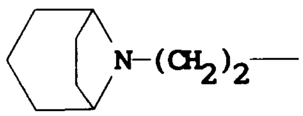

as well as its salts with mineral or organic acids, characterized in that it consists in reacting an amine of the formula

$H_2N\text{-}R_4$

in which $R_4$ is as defined above with a 3-chloropyridazine of the formula

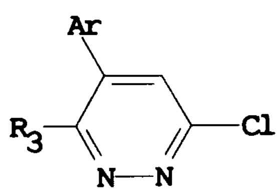

(II)

in which Ar and $R_3$ are as defined above, and, if desired, in converting the resulting compound to a salt with a mineral or organic acid.

**2.** Method according to claim 1, characterized in that an amine of formula $H_2N\text{-}R_4$ is used in which $R_4$ represents a group

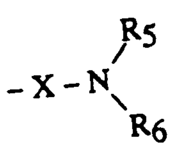

in which

- X represents
  . either a group $-(CH_2)n-$, in which n is an integer from 1 to 3;

. or a group

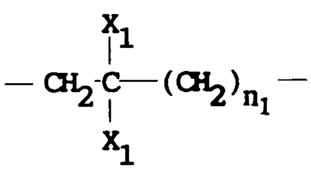

in which $X_1$ is a alkyl group which is lower than $C_3$ when $n_1 = 0$, or a methyl group when $n_1 = 1$;

- $R_5$ and $R_6$ are as defined in claim 1.

**3.** Method according to claim 1, characterized in that an amine of formula $H_2N-R_4$ is used in which $R_4$ represents a group

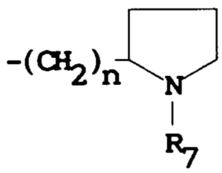

in which n is an integer from 1 to 3 and $R_7$ is a methyl or ethyl group.

**4.** Method for obtaining the compounds of formula:

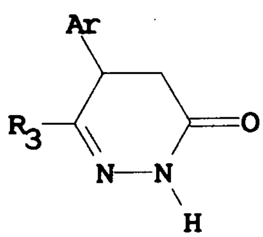

(IV)

in which:

- Ar is a phenyl group of the formula

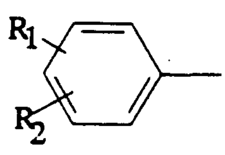

a pyridyl group or a thienyl group;
- $R_1$ and $R_2$ are each independently hydrogen, a halogen atom, a hydroxyl group, a $C_1$-$C_4$ alkyl group, a $C_1$-$C_4$ alkoxy or the trifluoromethyl group;
- $R_3$ is a linear or branched $C_1$-$C_5$ alkyl, a group $-CH_2-C_6H_5$ or a group $-CH_2-CH_2-C_6H_5$;

with the exception of the compound (IV) in which $R_1 = R_2 = H$ and $R_3 = CH_3$; characterized in that it consists in cyclising the ketone derivative of the formula:

$Ar-CH_2-CO-R_3$

in which Ar ad $R_3$ are as defined above by reaction with ethyl bromoacetate in a alcoholic solvent at room temperature, followed by reaction with hydrazine hydrate under the action of heat.

**5.** Method for obtaining the compounds of formula:

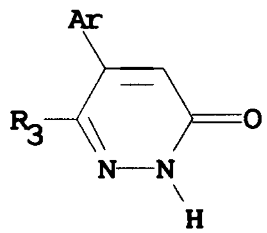

(III)

in which:

- Ar is a phenyl group of the formula

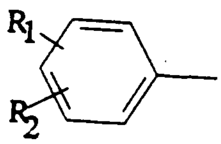

a pyridyl group or a thienyl group;
- $R_1$ and $R_2$ are each independently hydrogen, a halogen atom, a hydroxyl group, a $C_1$-$C_4$ alkyl group, a $C_1$-$C_4$ alkoxy or the trifluoromethyl group;
- $R_3$ is a linear or branched $C_1$-$C_5$ alkyl, a group $-CH_2-C_6H_5$ or a group $-CH_2-CH_2-C_6H_5$;

with the exception of the compound (III) in which $R_1 = R_2 = H$ ad $R_3 = CH_3$; characterized in that it consists in dehydrogenizing the compound of formula IV:

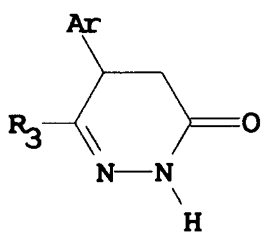

(IV)

in which Ar and $R_3$ are as defined above by reaction with bromine in acetic acid.

**6.** Method for obtaining the compounds of formula:

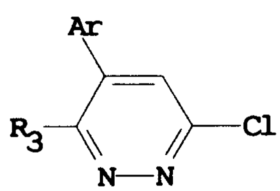

(II)

in which:

- Ar is a phenyl group of the formula

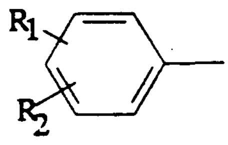

a pyridyl group or a thienyl group;
- $R_1$ and $R_2$ are each independently hydrogen, a halogen atom, a hydroxyl group, a $C_1$-$C_4$ alkyl group, a $C_1$-$C_4$ alkoxy or the trifluoromethyl group;
- $R_3$ is a linear or branched $C_1$-$C_5$ alkyl, a group $-CH_2-C_6H_5$ or a group $-CH_2-CH_2-C_6H_5$;

with the exception of the compound (II) in which $R_1 = R_2 = H$ and $R_3 = CH_3$; characterized in that it consists in reacting a compound of formula:

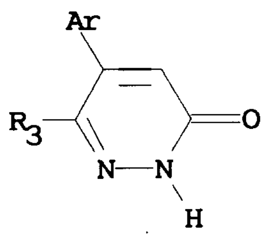

(III)

in which Ar and $R_3$ are as defined above with excess phosphorus oxychloride under the action of heat, without a solvent or in the presence of an inert solvent such as acetonitrile.

7. Method of preparing a pharmaceutical composition characterized in that a compound prepared according to the method of any one of claims 1 to 3 is mixed, as the active principle, with a pharmaceutically acceptable vehicle.

**Claims for the following Contracting State : GR**

1. Compound of the formula

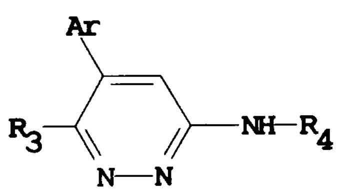

(I)

in which
- Ar is a phenyl group of the formula

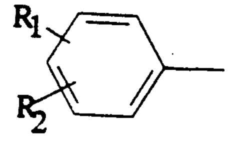

a pyridyl group or a thienyl group;
- $R_1$ and $R_2$ are each independently hydrogen, a halogen atom, a hydroxyl group, a $C_1$-$C_4$ alkyl group, a $C_1$-$C_4$ alkoxy or the trifluoromethyl group;
- $R_3$ is a linear or branched $C_1$-$C_5$ alkyl, a group $-CH_2-C_6H_5$ or a group $-CH_2-CH_2-C_6H_5$;

- $R_4$ is the group

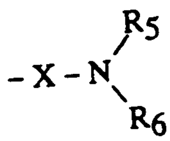

in which:

. X is a linear or branched $C_1$-$C_6$ alkylene;

. $R_5$ and $R_6$ are each independently hydrogen or a linear or branched $C_1$-$C_4$ alkyl or form, with the nitrogen atom to which they are bonded, a heterocycle selected from pyrrolidine, morpholine or piperidine

or $R_4$ is a group selected from:

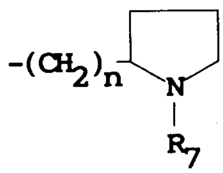

in which n is an integer from 1 to 3 and $R_7$ is hydrogen or a $C_1$-$C_4$ alkyl group;

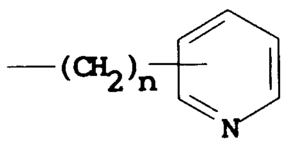

in which n is as defined above

or

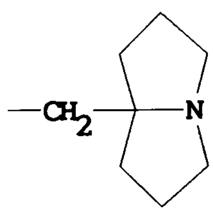

or

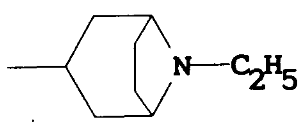

or

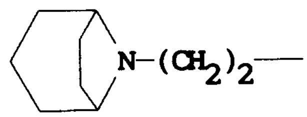

as well as its salts with mineral or organic acids.

**2.** Compound according to claim 1, characterized in that $R_4$ represents the group

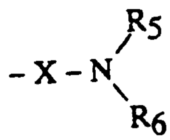

in which
- X represents
  - . either a group $-(CH_2)n-$, in which n is an integer from 1 to 3;
  - . or a group

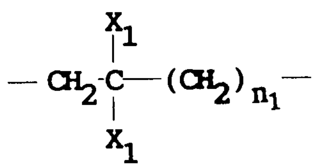

in which $X_1$ is a alkyl group which is lower than $C_3$ when $n_1 = 0$, or a methyl group when $n_1 = 1$;
- $R_5$ and $R_6$ are as defined in claim 1.

**3.** Compound according to claim 1, characterized in that $R_7$ is a methyl or ethyl group.

**4.** Intermediate compound for the preparation of compound (I) according to claim 1, of the formula:

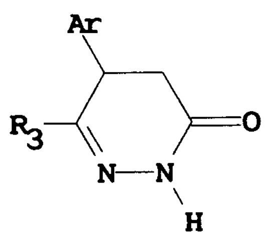

(IV)

in which Ar and $R_3$ are as defined in claim 1, with the exception of the compound (IV) in which $R_1 = R_2 = H$ and $R_3 = CH_3$.

5. Intermediate compound for the preparation of compound (I) according to claim 1, of the formula:

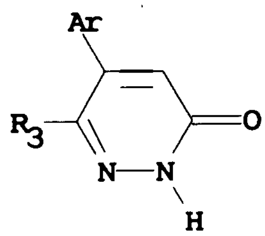

(III)

in which Ar and $R_3$ are as defined in claim 1, with the exception of the compound (III) in which $R_1$ = $R_2$ = H and $R_3$ = $CH_3$.

6. Intermediate compound for the preparation of compound (I) according to claim 1, of the formula:

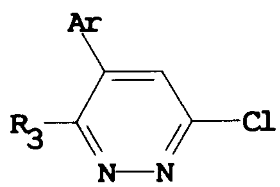

(II)

in which Ar and $R_3$ are as defined in claim 1, with the exception of the compound (II) in which $R_1$ = $R_2$ = H and $R_3$ = $CH_3$.

7. Method of preparing a compound according to claim 1, characterized in that an amine of the formula

$H_2N-R_4$

in which $R_4$ is as defined in claim 1, is reacted with a 3-chloropyridazine derivative of the formula

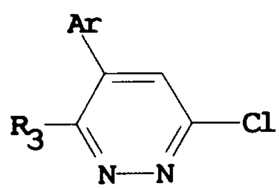

(II)

in which Ar and $R_3$ are as defined in claim 1, ad, if desired, the resulting compound is converted to a salt with a mineral or organic acid.

8. Method of preparing a pharmaceutical composition, characterized in that a compound according to any one of claims 1 to 3 is mixed, as the active principle, with a pharmaceutically acceptable vehicle.

**Patentansprüche**
**Patentansprüche für folgende Vertragsstaaten : AT, BE, CH, DE, DK, FR, GB, IT, LI, LU, NL, SE**

1. Verbindungen der Formel

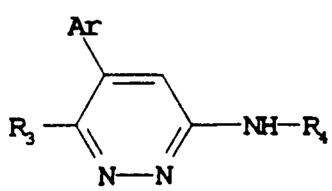

(I),

in der bedeuten:

- Ar eine Phenylgruppe der Formel

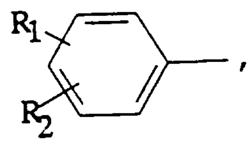

worin $R_1$ und $R_2$ unabhängig Wasserstoff, Halogen, Hydroxy, $C_{1-4}$-Alkyl, $C_{1-4}$-Alkoxy oder Trifluormethyl darstellen, Pyridyl oder Thienyl,

- $R_3$ geradkettiges oder verzweigtes $C_{1-5}$-Alkyl, eine Gruppe $-CH_2-C_6H_5$ oder eine Gruppe $-CH_2-CH_2-C_6H_5$, und
- $R_4$ eine Gruppe

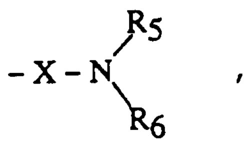

in der

- X ein geradkettiges oder verzweigtes $C_{1-6}$-Alkylen darstellt und
- $R_5$ und $R_6$ unabhängig Wasserstoff oder geradkettiges oder verzweigtes $C_{1-4}$-Alkyl bedeuten oder mit dem Stickstoffatom, an das sie gebunden sind, einen unter Pyrrolidin, Morpholin und Piperidin ausgewählten Heterocyclus bilden,

oder eine Gruppe, die ausgewählt ist unter

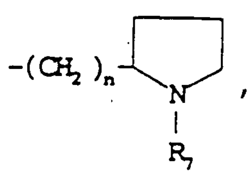

worin n eine ganze Zahl von 1 bis 3 und $R_7$ Wasserstoff oder $C_{1-4}$-Alkyl bedeuten,

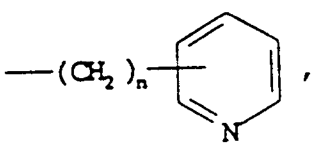

worin n dasselbe wie oben bedeutet,

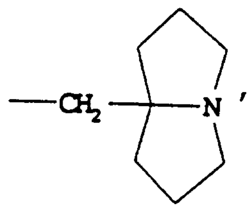

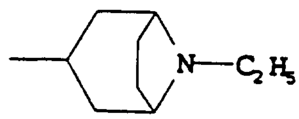

und

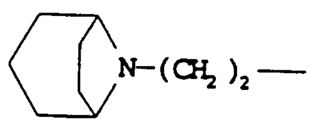

sowie ihre Salze mit anorganischen und organischen Säuren.

**2.** Verbindungen nach Anspruch 1, dadurch gekennzeichnet, daß $R_4$ eine Gruppe

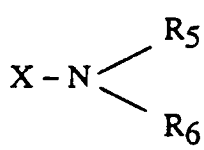

darstellt, worin bedeuten:

- X entweder eine Gruppe $-(CH_2)_n-$, in der n eine ganze Zahl von 1 bis 3 ist, oder eine Gruppe

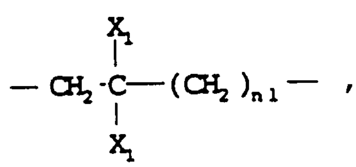

in der $X_1$ für $n_1 = 0$ eine Alkylgruppe mit weniger als 3 C-Atomen und für $n_1 = 1$ Methyl darstellt,
und

- $R_5$ und $R_6$ dasselbe wie in Anspruch 1.

**3.** Verbindungen nach Anspruch 1, dadurch gekennzeichnet, daß $R_7$ Methyl oder Ethyl ist.

4. Zwischenverbindungen zur Herstellung der Verbindungen (I) nach Anspruch 1 der Formel

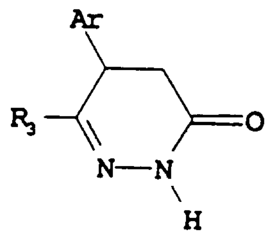

(IV),

in der Ar und $R_3$ dasselbe wie in Anspruch 1 bedeuten, wobei die Verbindung (IV) ausgenommen ist, für die $R_1 = R_2 = H$ und $R_3 = CH_3$ sind.

5. Zwischenverbindungen zur Herstellung von Verbindungen (I) nach Anspruch 1 der Formel

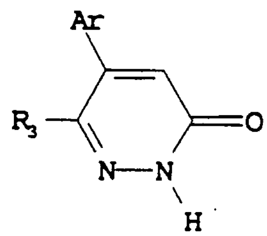

(III),

in der Ar und $R_3$ dasselbe wie in Anspruch 1 bedeuten, wobei die Verbindung (III) ausgenommen ist, für die $R_1 = R_2 = H$ und $R_3 = CH_3$ sind.

6. Zwischenverbindungen zur Herstellung der Verbindungen (I) nach Anspruch 1 der Formel

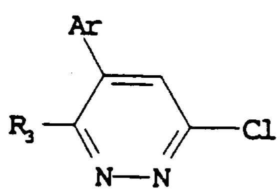

(II),

in der Ar und $R_3$ dasselbe wie in Anspruch 1 bedeuten, wobei die Verbindung (II) ausgenommen ist, für die $R_1 = R_2 = H$ und $R_3 = CH_3$ sind.

7. Verfahren zur Herstellung der Verbindungen nach Anspruch 1, dadurch gekennzeichnet, daß ein Amin der Formel

$H_2N\text{-}R_4$,

in der $R_4$ dasselbe wie in Anspruch 1 bedeutet, mit einem 3-Chlorpyridazin der Formel

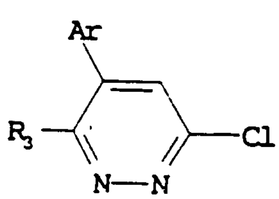

(II),

in der Ar und $R_3$ dasselbe wie in Anspruch 1 bedeuten, zur Reaktion gebracht wird, und daß die so hergestellten Verbindungen ggf. mit einer anorganischen oder organischen Säure in Salze übergeführt werden.

8. Pharmazeutische Zusammensetzungen, die als Wirkstoff eine der Verbindungen der Formel (I) nach einem der Ansprüche 1 bis 3 enthalten.

9. Pharmazeutische Zusammensetzungen nach Anspruch 8 in Form von Dosiereinheiten, in denen der Wirkstoff mit mindestens einem pharmazeutisch akzeptablen Excipiens gemischt ist.

**Patentansprüche für folgenden Vertragsstaat : ES**

1. Verfahren zur Herstellung von 6-Alkylpyridazinen der Formel

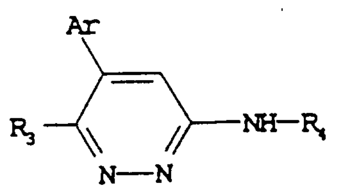

(I),

in der bedeuten:

- Ar eine Phenylgruppe der Formel

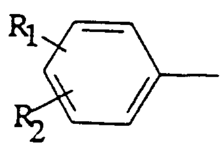

,

  worin $R_1$ und $R_2$ unabhängig Wasserstoff, Halogen, Hydroxy, $C_{1-4}$-Alkyl, $C_{1-4}$-Alkoxy oder Trifluormethyl darstellen, Pyridyl oder Thienyl,
- $R_3$ geradkettiges oder verzweigtes $C_{1-5}$-Alkyl, eine Gruppe $-CH_2-C_6H_5$ oder eine Gruppe $-CH_2-CH_2-C_6H_5$,
  und
- $R_4$ eine Gruppe

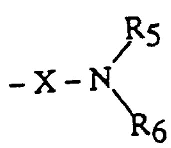

,

  in der
  - X ein geradkettiges oder verzweigtes $C_{1-6}$-Alkylen darstellt und
  - $R_5$ und $R_6$ unabhängig Wasserstoff oder geradkettiges oder verzweigtes $C_{1-4}$-Alkyl bedeuten oder mit dem Stickstoffatom, an das sie gebunden sind, einen unter Pyrrolidin, Morpholin und Piperidin ausgewählten Heterocyclus bilden,

oder eine Gruppe, die ausgewählt ist unter

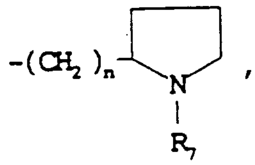

worin n eine ganze Zahl von 1 bis 3 und $R_7$ Wasserstoff oder $C_{1-4}$-Alkyl bedeuten,

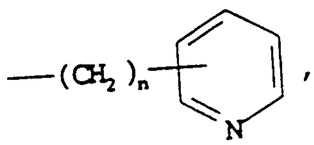

worin n dasselbe wie oben bedeutet,

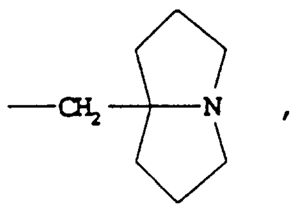

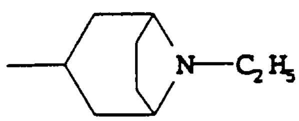

und

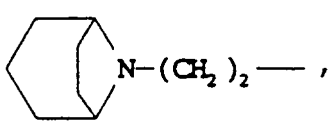

sowie ihre Salze mit anorganischen und organischen Säuren, dadurch gekennzeichnet, daß ein Amin der Formel

$H_2N$-$R_4$,

in der $R_4$ dasselbe wie oben bedeutet, mit einem Derivat von 3-Chlorpyridazin der Formel

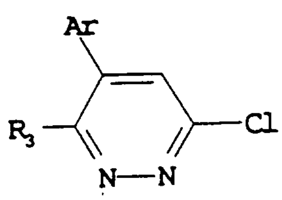

(II),

in der Ar und $R_3$ dasselbe wie oben bedeuten,
zur Reaktion gebracht wird,

und die so erhaltenen Verbindungen ggf. mit einer anorganischen oder organischen Säure in Salze übergeführt werden.

**2.** Verfahren nach Anspruch 1, dadurch gekennzeichnet, daß ein Amin der Formel $H_2N$-$R_4$ verwendet wird, in der $R_4$ die Gruppe

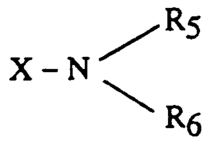

darstellt, worin bedeuten:

- X eine Gruppe $-(CH_2)_n-$, worin n eine ganze Zahl von 1 bis 3 ist, oder eine Gruppe

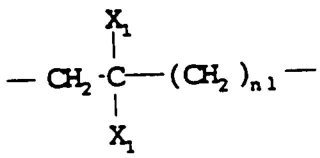

,

  in der $X_1$ für $n_1 = 0$ eine Alkylgruppe mit weniger als 3 C-Atomen und für $n_1 = 1$ Methyl darstellt,
  und
- $R_5$ und $R_6$ dasselbe wie in Anspruch 1.

**3.** Verfahren nach Anspruch 1, dadurch gekennzeichnet, daß ein Amin der Formel $H_2N$-$R_4$ verwendet wird, in der $R_4$ eine Gruppe

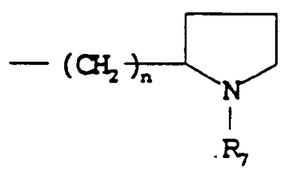

darstellt, worin n eine ganze Zahl von 1 bis 3 und $R_7$ Methyl oder Ethyl bedeuten.

**4.** Verfahren zur Herstellung von Verbindungen der Formel

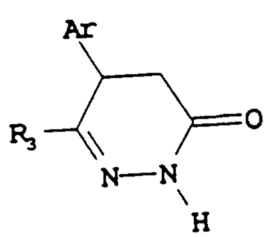

(IV) ,

in der bedeuten:

- Ar eine Phenylgruppe der Formel

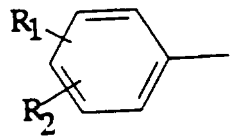

worin $R_1$ und $R_2$ unabhängig Wasserstoff, Halogen, Hydroxy, $C_{1-4}$-Alkyl, $C_{1-4}$-Alkoxy oder Trifluormethyl darstellen, Pyridyl oder Thienyl, und

- $R_3$ geradkettiges oder verzweigtes $C_{1-5}$-Alkyl, eine Gruppe $-CH_2-C_6H_5$ oder eine Gruppe $-CH_2-CH_2-C_6H_5$,

wobei die Verbindung (IV) ausgenommen ist, für die $R_1 = R_2 = H$ und $R_3 = CH_3$ sind, dadurch gekennzeichnet, daß ein Ketonderivat der Formel

$Ar-CH_2-CO-R_3$,

in der Ar und $R_3$ dasselbe wie oben bedeuten, durch Reaktion mit Ethylbromacetat in einem alkoholischen Lösungsmittel bei Raumtemperatur und anschließende Reaktion mit Hydrazinhydrat in der Hitze, cyclisiert wird.

**5.** Verfahren zur Herstellung von Verbindungen der Formel

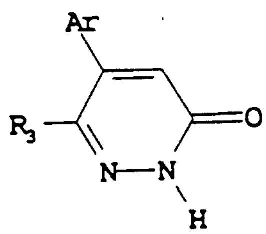

(III),

in der bedeuten:

- Ar eine Phenylgruppe der Formel

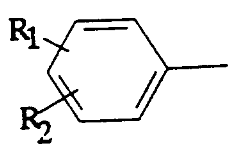

wobei $R_1$ und $R_2$ unabhängig Wasserstoff, Halogen, Hydroxy, $C_{1-4}$-Alkyl, $C_{1-4}$-Alkoxy oder Trifluormethyl darstellen, Pyridyl oder Thienyl, und

- $R_3$ geradkettiges oder verzweigtes $C_{1-5}$-Alkyl, eine Gruppe $-CH_2-C_6H_5$ oder eine Gruppe $-CH_2-CH_2-C_6H_5$,

wobei die Verbindung (III) ausgenommen ist, für die $R_1 = R_2 = H$ und $R_3 = CH_3$ sind, dadurch gekennzeichnet, daß eine Verbindung der Formel IV

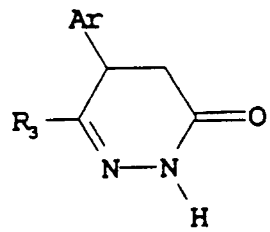 (IV),

in der Ar und $R_3$ dasselbe wie oben bedeuten, durch Einwirkung von Brom in Essigsäure dehydrogeniert wird.

**6.** Verfahren zur Herstellung von Verbindungen der Formel

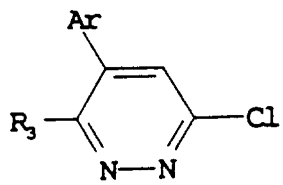 (II),

in der bedeuten:

- Ar eine Phenylgruppe der Formel

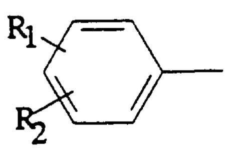 ,

worin $R_1$ und $R_2$ unabhängig Wasserstoff, Halogen, Hydroxy, $C_{1-4}$-Alkyl, $C_{1-4}$-Alkoxy oder Trifluormethyl darstellen, Pyridyl oder Thienyl,
und

- $R_3$ geradkettiges oder verzweigtes $C_{1-5}$-Alkyl, eine Gruppe $-CH_2-C_6H_5$ oder eine Gruppe $-CH_2-CH_2-C_6H_5$,

wobei die Verbindung (II) ausgenommen ist, für die $R_1 = R_2 = H$ und $R_3 = CH_3$ sind,
dadurch gekennzeichnet, daß eine Verbindung der Formel

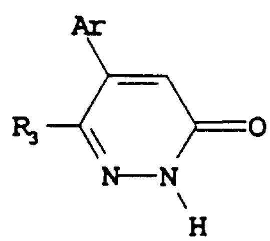 (III),

in der Ar und $R_3$ dasselbe wie oben bedeuten, mit einem Überschuß an Phosphoroxidchlorid in der Hitze ohne Lösungsmittel oder in Gegenwart eines inerten Lösungsmittels, wie Acetonitril, umgesetzt wird.

**7.** Verfahren zur Herstellung pharmazeutischer Zusammensetzungen, dadurch gekennzeichnet, daß eine nach dem Verfahren nach einem der Ansprüche 1 bis 3 hergestellte Verbindung als Wirkstoff mit einem pharmazeutisch akzeptablen Vehikel gemischt wird.

**Patentansprüche für folgenden Vertragsstaat : GR**

**1.** Verbindungen der Formel

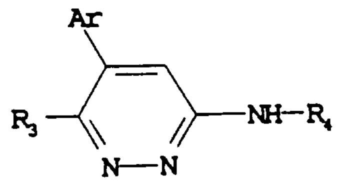

(I),

in der bedeuten:

- Ar eine Phenylgruppe der Formel

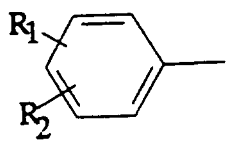

,

worin $R_1$ und $R_2$ unabhängig Wasserstoff, Halogen, Hydroxy, $C_{1-4}$-Alkyl, $C_{1-4}$-Alkoxy oder Trifluormethyl darstellen, Pyridyl oder Thienyl,
- $R_3$ geradkettiges oder verzweigtes $C_{1-5}$-Alkyl, eine Gruppe $-CH_2-C_6H_5$ oder eine Gruppe $-CH_2-CH_2-C_6H_5$,
und
- $R_4$ eine Gruppe

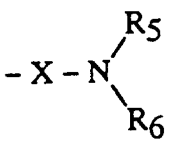

,

in der
- X ein geradkettiges oder verzweigtes $C_{1-6}$-Alkylen darstellt und
- $R_5$ und $R_6$ unabhängig Wasserstoff, oder geradkettiges oder verzweigtes $C_{1-4}$-Alkyl bedeuten oder mit dem Stickstoffatom, an das sie gebunden sind, einen unter Pyrrolidin, Morpholin und Piperidin ausgewählten Heterocyclus bilden,
oder eine Gruppe, die ausgewählt ist unter

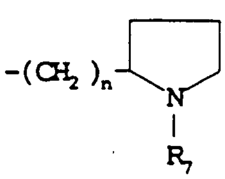

,

worin n eine ganze Zahl von 1 bis 3 und $R_7$ Wasserstoff oder $C_{1-4}$-Alkyl bedeuten,

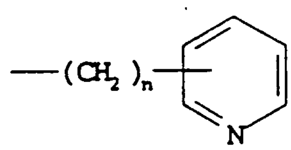

,

worin n dasselbe wie oben bedeutet,

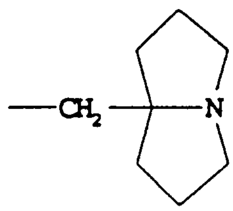

,

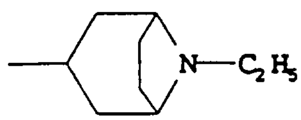

und

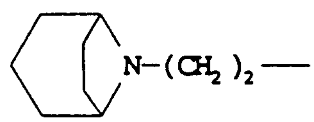

,

sowie ihre Salze mit anorganischen und organischen Säuren.

**2.** Verbindungen nach Anspruch 1, dadurch gekennzeichnet, daß $R_4$ eine Gruppe

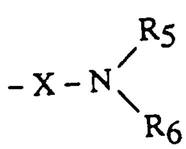

darstellt, worin bedeuten:

- X entweder eine Gruppe $-(CH_2)_n-$, in der n eine ganze Zahl von 1 bis 3 ist, oder eine Gruppe

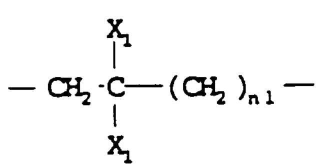

,

in der $X_1$ für $n_1 = 0$ eine Alkylgruppe mit weniger als 3 C-Atomen und für $n_1 = 1$ Methyl darstellt,
und

- $R_5$ und $R_6$ dasselbe wie in Anspruch 1.

3. Verbindungen nach Anspruch 1, dadurch gekennzeichnet, daß $R_7$ Methyl oder Ethyl ist.

4. Zwischenverbindungen zur Herstellung der Verbindungen (I) nach Anspruch 1 der Formel

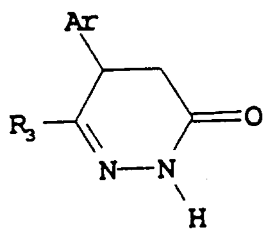

(IV) ,

in der Ar und $R_3$ dasselbe wie in Anspruch 1 bedeuten, wobei die Verbindung (IV) ausgenommen ist, für die $R_1$ = $R_2$ = H und $R_3$ = $CH_3$ sind.

5. Zwischenverbindungen zur Herstellung von Verbindungen (I) nach Anspruch 1 der Formel

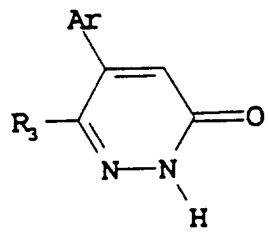

(III),

in der Ar und $R_3$ dasselbe wie in Anspruch 1 bedeuten, wobei die Verbindung (III) ausgenommen ist, für die $R_1$ = $R_2$ = H und $R_3$ = $CH_3$ sind.

6. Zwischenverbindungen zur Herstellung von Verbindungen (I) nach Anspruch 1 der Formel

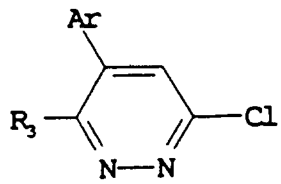

(II) ,

in der Ar und $R_3$ dasselbe wie in Anspruch 1 bedeuten, wobei die Verbindung (II) ausgenommen ist, für die $R_1$ = $R_2$ = H und $R_3$ = $CH_3$ sind.

7. Verfahren zur Herstellung der Verbindungen nach Anspruch 1, dadurch gekennzeichnet, daß ein Amin der Formel

$H_2N\text{-}R_4$,

in der $R_4$ dasselbe wie in Anspruch 1 bedeutet, mit einem 3-Chlorpyridazin der Formel

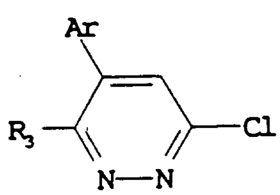

(II) ,

in der Ar und $R_3$ dasselbe wie in Anspruch 1 bedeuten, zur Reaktion gebracht wird, und daß die so hergestellten Verbindungen ggf. mit einer anorganischen oder organischen Säure in Salze übergeführt werden.

**8.** Verfahren zur Herstellung pharmazeutischer Zusammensetzungen, dadurch gekennzeichnet, daß als Wirkstoff eine Verbindung nach einem der Ansprüche 1 bis 3 mit einem pharmazeutisch akzeptablen Vehikel gemischt wird.